# EUROPÄISCHE PATENTSCHRIFT

(11) **EP 3 412 283 B1**
(45) Veröffentlichungstag und Bekanntmachung des Hinweises auf die Patenterteilung: **05.08.2026**
(21) Anmeldenummer: 18171470.0
(22) Anmeldetag: 09.05.2018
(51) Int. Cl.: A61K 9/70, A61K 31/485, A61P 25/04

(54) **OVERTAPE FÜR EIN TRANSDERMALES DARREICHUNGSSYSTEM**
OVERTAPE FOR A TRANSDERMAL DELIVERY SYSTEM
BANDE DE RECOUVREMENT POUR UN SYSTÈME D'ADMINISTRATION TRANSDERMIQUE

(30) Priorität: 07.06.2017 DE 102017112543
(43) Veröffentlichungstag der Anmeldung: 12.12.2018
(73) Patentinhaber: AdhexPharma SAS, 75016 Paris (FR)
(72) Erfinder: Rodler, Stefanie, 83626 Valley (DE)
(74) Vertreter: Beckord & Niedlich Patentanwälte PartG mbB

(56) Entgegenhaltungen:
- WO-A1-2012/065740
- DE-A1- 19 738 855

## Beschreibung

Die vorliegende Erfindung betrifft ein Overtape für ein transdermales Darreichungssystem, wobei das Overtape eine wirkstofffreie haftklebende Matrix und eine Rückschicht umfasst, wobei die wirkstofffreie haftklebende Matrix zumindest ein quervernetztes Polymer und zumindest ein nicht-quervernetztes Polymer umfasst, sowie ein transdermales Darreichungssystem mit dem erfindungsgemäßen Overtape. Des Weiteren betrifft die vorliegende Erfindung ein Verfahren zur Herstellung des erfindungsgemäßen Overtapes und des TDS mit dem erfindungsgemäßen Overtape sowie deren Verwendung.

Als Overtape wird im Allgemeinen ein einseitig klebender Klebematerialverbund aus einer Rückschicht und einer klebenden Schicht verstanden. Overtapes finden häufig Anwendung als Klebehilfe für nicht ausreichend klebende Materialien. Ein Overtape ist üblicherweise größer als das zu fixierende Material ausgestaltet und wird so aufgebracht, dass das Overtape mit der klebenden Seite auf dem zu fixierenden Material angeordnet ist und dieses umschließt.

Aufgrund der oben genannten Eigenschaften finden Overtapes häufig Verwendung zur Fixierung transdermaler Darreichungssysteme (TDS, *syn.* transdermale therapeutische Systeme, TTS), welche dem Verabreichen von Arzneistoffen durch die Haut eines Patienten in dessen Blutkreislauf dienen. Der Einsatz eines Overtapes ist insbesondere bei TDS sinnvoll, welche eine wirkstoffhaltige Matrix enthalten, die nicht oder nicht in ausreichendem Maße haftklebend ist, da so ein sicheres Anbringen des TDS auf der Haut ermöglicht und/oder ein vorzeitiges Ablösen des TDS verhindert wird. Solche Overtapes können dabei zunächst separat von dem TDS vorliegen und erst bei Applikation des TDS auf der Haut auf dieses aufgebracht werden. Jedoch kann ein Overtape auch schon auf einem TDS aufgebracht sein. Im Rahmen dieser Anmeldung ist der Begriff TDS bzw. "transdermales Darreichungssystem" so definiert, dass es ein Overtape umfasst. Der Teil des TDS, welcher unter anderem die wirkstoffhaltige Matrix umfasst, wird in diesem Rahmen als "TDS-Kern" bezeichnet, auch wenn ein solcher TDS-Kern natürlich bereits selber ohne Overtape als TDS eingesetzt werden könnte.

Die Umschließung des zu fixierenden Materials mit dem Overtape ermöglicht eine gute Fixierung auf der Haut des Anwenders über die gesamte Fläche des TDS. So kann ein guter Kontakt des TDS mit der Haut und damit eine kontinuierliche, kontrollierte Wirkstoffabgabe über den gesamten Applikationszeitraum gewährleistet werden.

Es hat sich gezeigt, dass die Haftung von Overtapes besonders unter Einwirkung mechanischer oder thermischer Belastungen bzw. bei Inkontaktbringen mit Schweiß und/oder Feuchtigkeit häufig nicht ausreicht, da diese beispielsweise auf der Haut verrutschen und sich das damit fixierte wirkstoffhaltige Material in manchen Fällen vollständig von der Haut ablöst, bevor die vorbestimmte Therapiedauer erreicht ist. Dies ist insbesondere wichtig für TDS, welche für eine Tragedauer von mehreren Tagen vorgesehen sind, da sich durch Unzulänglichkeiten der Klebeeigenschaften des Overtapes die Auflagefläche des fixierten, wirkstoffhaltigen Materials verändert und gegebenenfalls der therapeutische Effekt ausbleibt. Die WO 2003 047 556 beschreibt ein transdermales therapeutisches System mit einer Rückschicht, einer mindestens einen pharmazeutischen Wirkstoff enthaltenden makroporösen Diffusionsmatrix und einem Klebemittel.

Die DE 1973 8855 A1 betrifft ein transdermales therapeutisches System umfassend eine wiederablösbare Schutzschicht, eine haftklebende Reservoirschicht und eine gegebenenfalls haftkleberbeschichtete Rückschicht mit einem unidirektional, vorzugsweise längselastischen Material mit einer Elastizität von mindestens 20%.

WO 2012/065740 A1 beschreibt ein transdermales Darreichungssystem mit einer wirkstoffhaltigen Matrix mit einer Mischung aus einem quervernetzten Polyacrylat und einem nicht-quervernetzten Polyacrylat.

Es ist daher die Aufgabe der vorliegenden Erfindung, ein Overtape vorzusehen, das eine gute Fixierung einer Auflagefläche bewirkt. Das erfindungsgemäße Overtape ist insbesondere für die Fixierung transdermaler Darreichungssysteme vorteilhaft.

Diese Aufgabe wird erfindungsgemäß durch ein Overtape gemäß Anspruch 1 gelöst, welches zumindest eine wirkstofffreie haftklebende Matrix und eine Rückschicht umfasst, wobei die wirkstofffreie haftklebende Matrix eine Mischung aus zumindest einem quervernetzten Polymer und zumindest einem nicht-quervernetzten Polymer umfasst. Des Weiteren wird die Aufgabe durch ein Verfahren zur Herstellung des erfindungsgemäßen Overtapes gemäß Anspruch 12 gelöst.

Die vorliegende Erfindung betrifft daher ein Overtape umfassend
(a) eine wirkstofffreie haftklebende Matrix und
(b) eine Rückschicht,

wobei die wirkstofffreie haftklebende Matrix eine Mischung aus zumindest einem quervernetzten Polymer und zumindest einem nicht-quervernetzten Polymer umfasst,
wobei das quervernetzte Polymer ausgewählt ist aus der Gruppe der Polyacrylate, insbesondere aus der Gruppe der Acrylat/Vinylacetat-Copolymere,
wobei das nicht-quervernetzte Polymer ausgewählt ist aus der Gruppe der Polyacrylate, insbesondere aus der Gruppe der Acrylat/Vinylacetat-Copolymere.

Das erfindungsgemäße Overtape gemäß Anspruch 1 weist vorteilhaft in einem Bereich, welcher mit der Haut in Berührung kommt, gute Hafteigenschaften auf und ist somit zur dauerhaften Fixierung von TDS auf der Haut geeignet.

In ihrer einfachsten Ausführungsform umfasst ein erfindungsgemäßes Overtape zumindest eine wirkstofffreie, haftklebende Matrix und eine darauf angeordnete Rückschicht, wobei die wirkstofffreie haftklebende Matrix eine Mischung aus zumindest einem quervernetzten Polymer und zumindest einem nicht-quervernetzten Polymer umfasst, wobei das quervernetzte Polymer ausgewählt ist aus der Gruppe der Polyacrylate, insbesondere aus der Gruppe der Acrylat/Vinylacetat-Copolymere und wobei das nicht-quervernetzte Polymer ausgewählt ist aus der Gruppe der Polyacrylate, insbesondere aus der Gruppe der Acrylat/Vinylacetat-Copolymere.

Gegebenenfalls kann die Mischung darüber hinaus auch ein Vernetzungsmittel enthalten, welches nicht mit einem Polymer vernetzt ist.

Die auf der wirkstofffreien haftklebenden Matrix angeordnete Rückschicht eines erfindungsgemäßen Overtapes besteht vorteilhaft aus einem folienartigem Material und/oder einem Gewebe oder Vlies.

Vorteilhaft handelt es sich bei der Mischung aus zumindest einem quervernetzten Polymer und zumindest einem nicht-quervernetzten Polymer um eine homogene Mischung.

Unter einem Polymer wird in der vorliegenden Patentanmeldung ein Homo- und/oder ein Heteropolymer verstanden, welches aus mehreren Monomeren besteht, die über kovalente Bindungen linear miteinander verbunden sind, wobei eine lineare Hauptkette ferner auch verzweigte Seitenketten aufweisen kann. Während Homopolymere eine kovalente Verkettung gleicher Monomere aufweisen, sind Heteropolymere (auch Copolymere genannt) aus unterschiedlichen Monomeren aufgebaut.

Neben der kovalenten Bindung können auch (induzierte) Dipol-Dipol-Kräfte, Dispersionskräfte und/oder Wasserstoffbrückenbindungen in Polymeren bzw. Copolymeren auftreten, die unter dem Begriff der Nebenvalenzbindungen zusammengefasst werden. Solche Nebenvalenzbindungen können zu den Anziehungskräften zwischen den Polymerketten beitragen, sind jedoch von einer Quervernetzung zu unterscheiden.

In der vorliegenden Patentanmeldung wird ein Polymer dann als quervernetztes Polymer bezeichnet, wenn das Polymer über kovalente, ionische und/oder koordinative Bindungen zwischen mindestens zwei Monomeren verschiedener Polymerketten quervernetzt ist. Die Quervernetzung wird gewöhnlich über sogenannte funktionelle Gruppen der Monomere vermittelt, wobei unter funktionellen Gruppen Atomgruppen verstanden werden, die maßgeblich für das Reaktionsverhalten eines Moleküls verantwortlich sind. Somit schließen quervernetzte Polymere selbstverständlich auch Polymere ein, bei welchen alle funktionellen Gruppen, die in einem quervernetzten Polymer vorliegen, an der Quervernetzung beteiligt sind. Es können jedoch auch funktionelle Gruppen in reaktiver, ungebundener Form in dem quervernetzten Polymer vorliegen.

Entsprechend wird ein Polymer als nicht-quervernetztes Polymer bezeichnet, wenn das Polymer nicht über funktionelle Gruppen quervernetzt ist, auch wenn es durchaus funktionelle Gruppen in reaktiver, ungebundener Form und Nebenvalenzbindungen aufweisen kann.

Das erfindungsgemäße Overtape kann vorteilhaft für ein transdermales Darreichungssystem (TDS) verwendet werden und so zu verbesserten Hafteigenschaften des TDS beitragen. Da zur Herstellung von wirkstoffhaltigen Matrices in TDS häufig Materialien verwendet werden, welche kaltfließende Eigenschaften aufweisen können und beispielsweise während der Lagerung zu fließen beginnen, hat die Umschließung des zu fixierenden Materials mit einem Overtape den weiteren Vorteil, dass ein seitliches Austreten von möglicherweise in dem Material enthaltenen Wirkstoffen verhindert wird. Besonders bei der Applikation von TDS, die hochwirksame Wirkstoffe beinhalten, wird damit der Gefahr einer ungewollten Kontamination von mit dem Material in Berührung kommenden Personen oder Gegenständen vorgebeugt. Außerdem wird dadurch ein möglicher Wirkstoffverlust verringert bzw. verhindert.

Ein erfindungsgemäßes Overtape ragt vorteilhaft auf allen Seiten über eine wirkstoffhaltige Matrix des TDS hinaus, sodass die wirkstoffhaltige Matrix mit deren Applikationsseite, d.h. mit der im Allgemeinen zum Anbringen auf der Haut vorgesehenen bzw. mit der Haut in Kontakt kommenden Seite des TDS, mit der Haut des Anwenders in Kontakt steht und von allen anderen Seiten mit dem Overtape umschlossen ist. So wird eine gute, kontinuierliche Wirkstoffpermeation gewährleistet. Im Folgenden wird wie erwähnt die wirkstoffhaltige Matrix des in der vorliegenden Patentanmeldung auch als TDS-Kern bezeichnet. Wie unten erläutert, kann ein solcher TDS-Kern neben der wirkstoffhaltigen Matrix auch eine optionale Trennschicht umfassen. Insofern ein erfindungsgemäßes Overtape und ein TDS-Kern (also ein herkömmliches transdermales Darreichungssystem ohne Overtape) separat vorliegen, kann das Overtape natürlich auch erst bei dessen Applikation in oben beschriebener Weise auf dem TDS-Kern angebracht werden.

Ein erfindungsgemäßes transdermales Darreichungssystem umfasst also zumindest
(a) das erfindungsgemäße Overtape,
(b) einen TDS-Kern umfassend eine wirkstoffhaltige Matrix und optional eine Trennschicht, sowie
(c) optional einen Release Liner.

Vorteilhaft ragt dabei das Overtape auf allen Seiten der wirkstoffhaltigen Matrix über die wirkstoffhaltige Matrix und/oder die optionale Trennschicht des TDS-Kerns hinaus.

Die vorliegende Erfindung betrifft ebenfalls die medizinische, tiermedizinische und/oder kosmetische Verwendung des erfindungsgemäßen Overtapes, insbesondere für ein transdermales Darreichungssystem.

Des Weiteren betrifft die vorliegende Erfindung ein Verfahren zur Herstellung eines erfindungsgemäßen Overtapes, wobei das Verfahren folgende Schritte umfasst:
(i) Bereitstellen zumindest einer wirkstofffreien haftklebenden Matrix enthaltend eine Mischung aus zumindest einem quervernetzten Polymer und einem nicht-quervernetzten Polymer,
(ii) Aufbringen einer Rückschicht auf die wirkstofffreie haftklebende Matrix zum Erhalt eines Overtape-Laminats,

wobei das quervernetzte Polymer ausgewählt ist aus der Gruppe der Polyacrylate, insbesondere aus der Gruppe der Acrylat/Vinylacetat-Copolymere,
wobei das nicht-quervernetzte Polymer ausgewählt ist aus der Gruppe der Polyacrylate, insbesondere aus der Gruppe der Acrylat/Vinylacetat-Copolymere.

Unter Bereitstellen wird dabei sowohl eine Herstellung vor Ort als auch ein Zuliefern einer wirkstofffreien haftklebenden Matrix verstanden. Hierbei kann die wirkstofffreie haftklebende Matrix bereits mit einer bedeckenden Schutzfolie versehen sein, welche im weiteren Verlauf der Herstellung in einem oder mehreren Herstellungsschritten durch eine alternative Schutzfolie ersetzt oder gänzlich entfernt werden kann.

Im Zuge des erfindungsgemäßen Verfahrens wird durch Aufbringen einer Rückschicht auf eine wirkstofffreie haftklebende Matrix ein Overtape-Laminat hergestellt. Unter einem Laminat kann dabei ein Wirkstoff oder ein Produkt verstanden werden, der bzw. das aus zwei oder mehreren flächig miteinander verklebten Schichten besteht. Im Folgenden wird der Begriff Overtape-Laminat mit Overtape gleichgesetzt.

Schließlich umfasst die vorliegende Erfindung ein Overtape, insbesondere für ein transdermales Darreichungssystem, welches nach dem vorstehend beschriebenen Verfahren erhältlich ist.

Prinzipiell können die in der wirkstofffreien Matrix des erfindungsgemäßen Overtapes enthaltenen funktionellen Gruppen unterschiedlich ausgebildet sein. Dabei können funktionelle Gruppen der Monomere eines quervernetzten bzw. nicht-quervernetzten Polymers beispielsweise aus Carboxy-, Hydroxy-, Allyl-, Amid-, Amin-, Isocyanat- und/oder Epoxygruppen ausgebildet sein. In einer bevorzugten Ausführungsform sind die funktionellen Gruppen eines quervernetzten Polymers jedoch aus Carboxy-, Hydroxy-, Amid-, Aminund/oder Epoxygruppen, insbesondere aus Carboxy-, Hydroxy- und Amingruppen, insbesondere bevorzugt aus Carboxygruppen, ausgewählt. In einer weiter bevorzugten Ausführungsform umfasst ein nicht-quervernetztes Polymer ebenfalls funktionelle Gruppen, welche aus Carboxy-, Hydroxy-, Allyl-, Amid-, Amin-, Isocyanat- und/oder Epoxygruppen, insbesondere aus Carboxy-, Hydroxy- und Amingruppen, insbesondere bevorzugt Carboxygruppen, ausgewählt sind.

Ferner lassen sich die Haftklebeeigenschaften des erfindungsgemäßen Overtapes durch den Gehalt der funktionellen Gruppen des zumindest einen quervernetzten bzw. nicht-quervernetzten Polymers in der wirkstofffreien haftklebenden Matrix anpassen. Vorzugsweise weist dabei das zumindest eine quervernetzte Polymer einen Gehalt an funktionellen Gruppen, insbesondere an Carboxygruppen, auf, der im Bereich von etwa 0,5 bis etwa 50 Mol-%, bevorzugt etwa 3 bis etwa 25 Mol-%, insbesondere im Bereich von etwa 5 bis etwa 11 Mol-%, liegt.

In einer besonders bevorzugten Ausführungsform weist auch das nicht-quervernetzte Polymer einen Gehalt an Carboxygruppen auf, der im Bereich von etwa 0,5 bis etwa 50 Mol-%, bevorzugt etwa 3 bis etwa 25 Mol-%, insbesondere im Bereich von etwa 5 bis etwa 11 Mol-%, liegt.

Die Quervernetzung zur Herstellung des zumindest einen quervernetzten Polymers kann prinzipiell durch Erwärmen, Altern oder Bestrahlen der Polymere mit beschleunigten Elektronen, Ultraviolett- und/oder Gammastrahlung und/oder durch Zugabe eines Additivs, das mit den eingeführten funktionellen Gruppen reagieren kann, erfolgen. Bevorzugt kann die Quervernetzung jedoch mittels Zugabe eines Vernetzungsmittels ausgelöst werden, welches bevorzugt zu einem nicht-quervernetzten Polymer bzw. nach einer Polymerisationsreaktion zum Reaktionsgemisch zugegeben wird. Die Zugabe kann allerdings auch schon während einer Polymerisationsreaktion erfolgen.

In einer bevorzugten Ausführungsform wird die Quervernetzung durch Zugabe eines Vernetzungsmittels ausgeführt, welches eine teilweise oder vollständige Vernetzung des Polymers über dessen funktionelle Gruppen bewirkt. Durch die Quervernetzung kann Einfluss auf die Haftklebeeigenschaften wie etwa Klebrigkeit, Schälfestigkeit, Zugfestigkeit und Kriechfestigkeit genommen und diese den Anforderungen entsprechend, insbesondere bezüglich der angestrebten Therapiedauer, angepasst werden.

Für die Herstellung eines quervernetzten Polymers werden die Monomere bzw. das Polymer zunächst mit einem Vernetzungsmittel versehen. In einem nächsten Schritt kann das quervernetzte Polymer mit einem nicht-quervernetzten Polymer gemischt werden.

Geeignete Vernetzungsmittel umfassen di- oder multivalente Metallionen, welche beispielsweise mit Carboxygruppen Metallcarboxylate bilden können. Die Metallionen werden normalerweise in Form von Metallchelaten angewandt, welche in organischen Lösungsmitteln löslich sind. Als bevorzugte Vernetzungsmittel können die folgenden Verbindungen genannt werden: Diphenyl-Methan-4-Diisocyanat, Hexamethylen-Diisocyanat, Titanium-Acetylacetonat, Aluminium-Acetylacetonat, Eisen-Acetylacetonat, Zink-Acetylacetonat, Magnesium-Acetylacetonat, Zirkonium-Acetylacetonat, 2-Ethyl-1,3-Hexanediol-Titanat, Tetra-Isooctyl-Titanat, Tetra-Nonyl-Titanat, polyfunktionale Propylen-Imin-Derivate, Ether-Derivate von Melamin-Formaldehyd-Harzen, hochmethylierte Urethan-Harze und Imino-MelaminHarze.

Ein besonders bevorzugtes Vernetzungsmittel ist jedoch Aluminium-Acetylacetonat und kann beispielsweise bei der Firma Merck (Deutschland) erworben werden.

Dabei weist das Vernetzungsmittel vorteilhaft einen Trockengewichtsanteil von unter 2 Gew.-%, bevorzugt etwa 1 Gew.-%, insbesondere etwa 0,4 Gew.-%, des Trockengewichtsanteils des quervernetzten Polymers auf.

Eine weitere Möglichkeit der Modifizierung der Haftklebeeigenschaften des erfindungsgemäßen Overtapes ist die Wahl der Monomere, aus welchen sich das zumindest eine quervernetzte bzw. das nicht-quervernetzte Polymer zusammensetzen. Bei der Auswahl der Monomere können die daraus resultierenden Polymere prinzipiell so entworfen werden, dass sie spezifische Eigenschaften aufweisen, d.h. eine günstige Lösungskapazität für einen Wirkstoff, eine gewünschte Beweglichkeit des Wirkstoffes in der Matrix sowie eine gewünschte Transferrate über die Haut. Vorteilhaft weisen Monomere, aus denen die oben genannten Polymere hergestellt werden können, etwa 2 bis etwa 15 Kohlenstoffatome, bevorzugt etwa 3 bis etwa 13 Kohlenstoffatome, insbesondere etwa 3 bis etwa 11 Kohlenstoffatome, auf.

Gemäß der vorliegenden Erfindung sind die Monomere des quervernetzten Polymers ausgewählt aus der Gruppe der Acrylate. In der vorliegenden Patentanmeldung wird unter einem Polyacrylat ein Polymer verstanden, welches gleiche oder verschiedene Monomer-Einheiten aufweist, wobei das oder die Monomere vorteilhaft aus der Gruppe ausgewählt sind von Acrylsäure, n-Butylacrylat, iso-Butylacrylat, Propylacrylat, Methylacrylat, 2-Ethylhexylacrylat, 2-Hydroxyethylacrylat und 2-Hydroxyethylmethacrylat.

In einer weiter bevorzugten Ausführungsform kann ein Polyacrylat-Polymer einen Gehalt an einem oder mehreren Monomeren aufweisen, welches bzw. welche zusätzlich zu den oben genannten ein oder mehreren Monomeren nicht aus der Gruppe der Acrylate ausgewählt sind. Vorteilhaft liegt dabei der Trockengewichtsanteil an Nicht-Acrylat-Monomeren am Gesamttrockengewicht der Monomere, die ein quervernetztes Polymer umfasst, bei maximal 52 Gew.-%. Bevorzugt liegt der Anteil an Nicht-Acrylat-Monomeren jedoch bei etwa 1 bis etwa 50 Gew.-%, besonders bevorzugt bei etwa 5 bis etwa 45 Gew.-%, insbesondere bei etwa 10 bis etwa 43 Gew.-%.

Solche Nicht-Acrylat-Monomere sind vorzugsweise ausgewählt aus der Gruppe von Styrol, Vinylacetat und/oder Tetramethylsuccinnitril, bevorzugt aus der Gruppe von Vinylacetat und Tetramethylsuccinnitril, insbesondere aus der Gruppe von Vinylacetat. In einer besonders bevorzugten Ausführungsform weist ein quervernetztes Polymer einen Trockengewichtsanteil an Vinylacetat von 45% und einen Trockengewichtsanteil an Tetramethylsuccinnitril von etwa 5% am Gesamttrockengewicht der Monomere auf.

Gemäß der vorliegenden Erfindung ist auch das nicht-quervernetzte Polymer ausgewählt aus der Gruppe der Polyacrylate, wobei das oder die Monomere aus der Gruppe ausgewählt sind von Acrylsäure, n-Butylacrylat, iso-Butylacrylat, Propylacrylat, Methylacrylat, 2-Ethylhexylacrylat, 2-Hydroxyethylacrylat und 2-Hydroxyethylmethacrylat.

Dabei kann gemäß einer bevorzugten Ausführungsform auch das nicht-quervernetzte Polymer einen Gehalt an Nicht-Acrylat-Monomeren aufweisen. Der Trockengewichtsanteil an Nicht-Acrylat-Monomeren am Gesamttrockengewicht der Monomere, die ein nicht-quervernetztes Polymer umfasst, liegt vorteilhaft bei maximal 45 Gew.-%. Bevorzugt liegt der Anteil an Nicht-Acrylat-Monomeren jedoch bei etwa 1 bis etwa 40 Gew.-%, besonders bevorzugt bei etwa 5 bis etwa 38 Gew.-%, insbesondere bei etwa 10 bis etwa 35 Gew.-%.

Die Haftklebeeigenschaften der wirkstofffreien Matrix des erfindungsgemäßen Overtapes lassen sich ferner durch die Anordnung der Monomere in dem zumindest einen quervernetzten und dem zumindest einen nicht-quervernetzten Polymer modifizieren. Da diese Anordnung nur bei unterschiedlichen Monomeren eine Rolle spielt, werden hierbei speziell Copolymere je nach Anordnung ihrer Monomere in Klassen wie beispielsweise statistische, alternierende, Gradienten-, Pfropf- oder Block-Copolymere eingeteilt. Bevorzugt sind wiederum Polymere einer Anordnung, welche in Kombination mit dem jeweiligen Wirkstoff geeignete Eigenschaften aufweisen, d.h. eine günstige Lösungskapazität für den Wirkstoff, eine gewünschte Beweglichkeit des Wirkstoffes in der Matrix sowie eine gewünschte Transferrate über die Haut. Dabei haben sich statistische Copolymere, auch als Random-Copolymere bezeichnet, alternierende Copolymere und Pfropf-Copolymere als besonders geeignet für das erfindungsgemäße Overtape erwiesen.

Bevorzugte quervernetzte Polyacrylate können beispielsweise unter der Bezeichnung Durotak 87-2052 bei Henkel (Deutschland) erworben werden. Bevorzugte nicht-quervernetzte Polyacrylate sind beispielsweise unter dem Namen Durotak 87-2051 und 87-2353 bei Henkel (Deutschland) erhältlich.

Das Mischungsverhältnis aus zumindest einem quervernetzten Polymer und zumindest einem nicht-quervernetzten Polymer kann die haftklebenden Eigenschaften der wirkstofffreien haftklebenden Matrix eines erfindungsgemäßen Overtapes beeinflussen.

Dabei hat sich gezeigt, dass eine bevorzugte Ausführungsform ein Verhältnis der Gewichte des quervernetzten Polymers zu dem nicht-quervernetzten Polymer in der getrockneten Matrix in einem Bereich von etwa 1:4 bis etwa 4:1, besonders bevorzugt in einem Bereich von etwa 1:2 bis etwa 2:1, insbesondere in einem Bereich von etwa 1:1,5 bis etwa 1,5:1, insbesondere bevorzugt in einem Bereich von etwa 1:1, aufweist. Dabei bestimmt der Dividend der angegebenen Verhältnisse jeweils den zur Mischung zuzugebenden Gewichtsteil des quervernetzten Polymers und der Divisor den zur Mischung zuzugebenden Gewichtsteil des nicht-quervernetzten Polymers.

Insofern in der wirkstofffreien haftklebenden Matrix mehr als ein quervernetztes und/oder mehr als ein nicht-quervernetztes Polymer vorliegen, bezieht sich das Verhältnis von quervernetztem Polymer zu nicht-quervernetztem Polymer auf den Trockengewichtsanteil von allen quervernetzten Polymeren zum Trockengewichtsanteil von allen nicht-quervernetzten Polymeren. Dieses Verhältnis liegt bevorzugt in einem Bereich von etwa 1:4 bis etwa 4:1, besonders bevorzugt in einem Bereich von etwa 1:2 bis etwa 2:1, insbesondere in einem Bereich von etwa 1:1,5 bis etwa 1,5:1, insbesondere bevorzugt in einem Bereich von etwa 1:1. Bevorzugt umfasst ein erfindungsgemäßes Overtape jedoch lediglich ein quervernetztes Polymers und ein nicht-quervernetztes Polymer.

Wie oben beschrieben, kann das erfindungsgemäße Overtape vorteilhaft für ein transdermales Darreichungssystem verwendet werden, wobei das TDS einen TDS-Kern mit einer wirkstoffhaltigen Matrix umfasst. Optional kann dabei der TDS-Kern bzw. die wirkstoffhaltige Matrix eine Trennschicht umfassen. Ferner kann die wirkstoffhaltige Matrix des TDS von einer Schutzfolie, einem sogenannten Release Liner, abgedeckt sein, welcher vor Applikation des TDS entfernt wird.

Dies gilt insbesondere für eine Verpackungsform, in der das Overtape bereits vor Applikation des TDS auf diesem aufgebracht ist. In diesem Fall liegt das TDS mit dem TDS-Kern und dem Overtape also bereits als eine Einheit in einer Verpackung vor und kann so nach Entnahme aus der Verpackung als eine Einheit auf der Haut appliziert werden.

Das erfindungsgemäße Overtape kann aber beispielsweise auch als eigene Verpackungseinheit, also getrennt von dem TDS-Kern (bzw. einem einfachen, herkömmlichen TDS ohne eigenes, fest damit verbundenes Overtape) vorliegen, und erst nach oder bei Applikation des TDS-Kerns auf die Haut auf den TDS-Kern bzw. ein herkömmliches, einfaches TDS aufgebracht werden. Das erfindungsgemäße Overtape kann dabei auch eigenständig für verschiedene einfache, herkömmliche TDS (ohne integriertes Overtape) verwendet werden.

Prinzipiell können in allen oben genannten Darreichungsvarianten sowohl das erfindungsgemäße Overtape selbst als auch der TDS-Kern eine unterschiedliche Ausgestaltung bzw. Form aufweisen. So können sowohl das Overtape als auch der TDS-Kern beispielsweise als rechteckförmige, quadratische, runde, ovale oder vergleichbare Fläche ausgestaltet sein. Bevorzugt weist dabei eine rechteckförmige oder quadratische Fläche abgerundete Ecken auf. Insbesondere sind das erfindungsgemäße Overtape oder der TDS-Kern gleichermaßen als rechteckförmige, quadratische oder runde Fläche ausgestaltet, wenngleich das erfindungsgemäße Overtape oder der TDS-Kern auch in unterschiedlicher Form ausgestaltet sein können, wie beispielsweise das Overtape in Form einer runden Fläche und der TDS-Kern als rechteckförmige Fläche.

Um angemessene Haftklebeeigenschaften des TDS zu gewährleisten und das Auslaufen des Wirkstoffs aus der wirkstoffhaltigen Matrix zu minimieren, ragt das Overtape, wie oben beschrieben, über die wirkstoffhaltige Matrix bzw. den TDS-Kern hinaus. Dabei entspricht die über die wirkstoffhaltige Matrix hinausragende Fläche des Overtapes vorteilhaft zumindest dem 0,5-fachen, bevorzugt zumindest dem 0,7-fachen, insbesondere zumindest dem 0,8-fachen, der an der Haut aufliegenden Fläche einer wirkstoffhaltigen Matrix.

Wie eingangs erwähnt, weist ein erfindungsgemäßes Overtape eine Rückschicht auf, welche mit der wirkstofffreien haftklebenden Schicht ein Overtape-Laminat bildet. Vorteilhaft ist diese Rückschicht wirkstoffundurchlässig ausgestaltet und verhindert somit bei einer Verwendung für ein wirkstoffhaltiges TDS, insbesondere wenn der TDS-Kern eines solchen TDS (bzw. ein einfaches, herkömmliches TDS, für welches das Overtape verwendet werden soll) keine bzw. eine wirkstoffdurchlässige Trennschicht aufweist, dass Wirkstoff aus dem TDS austritt.

Geeignete Materialien für solche wirkstoffundurchlässige Rückschichten sind beispielsweise Polymere aus der Gruppe umfassend Polyolefine wie z. B. Polyethylene, Polypropylene oder Polybutylene, Olefin-Copolymerisate, Polyester und hiervon insbesondere Polyethylenterephthalate und/oder Polycarbonate, Co-Polyester, Polyamide, Co-Polyamide, Polyethylenoxide, Polyurethane, Polystyrole, Polyamide, Polyimide, Polyvinylacetate, Polyvinylchloride, Polyvinylidenchloride, Co-Polymerisate wie beispielsweise AcrylnitrilButadien-Styrol- Terpolymere, oder Ethylen-Vinylacetat-Copolymerisate und dergleichen genannt werden. Ein bevorzugtes Material eines Overtapes ist ausgewählt aus einem Polyolefin und/oder einem Polyester, besonders bevorzugt aus einer Polyolefin-Schaumfolie oder einem Polyester-Gewebe. Eine besonders vorteilhafte Polyolefin-Schaumfolie kann beispielsweise bei Sekisui Alveo (Luzern, Schweiz), ein vorteilhaftes Polyester-Gewebe kann bei Karl Otto Braun bezogen werden.

Es hat sich gezeigt, dass die Haftklebeeigenschaften eines Overtapes ferner durch die Dicke der wirkstofffreien haftklebenden Schichten beeinflusst werden können. Vorteilhaft beträgt die Schichtdicke der getrockneten wirkstofffreien haftklebenden Matrix weniger als 1 mm, bevorzugt liegt die Schichtdicke jedoch in einem Bereich von etwa 10 µm bis etwa 500 µm, besonders bevorzugt in einem Bereich von etwa 20 µm bis etwa 250 µm, insbesondere in einem Bereich von etwa 30 µm bis etwa 200 µm, gemessen jeweils ungestreckt.

Die wirkstoffhaltige Matrix eines vorteilhaften TDS kann prinzipiell haftklebend oder nichthaftklebend ausgestaltet sein. Dabei kann die wirkstoffhaltige Matrix-Schicht eines vorteilhaften TDS, welches nachfolgend auch als Pflaster bezeichnet wird, als feste bis halbfeste Matrix-Schicht ausgebildet sein und dabei ein Wirkstoffdepot bilden.

Die wirkstoffhaltige Matrix eines vorteilhaften TDS kann für solche Matrices prinzipiell bekannte Materialien aufweisen. Vorzugsweise umfasst die wirkstoffhaltige Matrix jedoch zumindest ein Polymer aus der Gruppe der Polyacrylate, wobei das zumindest eine Polyacrylat-Polymer ferner einen Gehalt an einem oder mehreren Monomeren aufweisen kann, welches bzw. welche nicht aus der Gruppe der Acrylate ausgewählt sind.

Wie vorstehend erläutert, ist in der wirkstoffhaltigen Matrix bevorzugter transdermaler Darreichungssysteme zumindest ein Wirkstoff enthalten. Dieser zumindest eine Wirkstoff ist, ohne beschränkend zu sein, ausgewählt aus der Klasse der Analgetika, Sympathomimetika, Sympatholytika, Parasympathomimetika, Dopaminagonisten, Hormone, Hormonantagonisten, Antidepressiva, Neuroleptika und/oder Antidementiva.

Nützliche Wirkstoffe umfassen, ohne dabei beschränkend zu sein, Opioid-Analgetika wie beispielsweise Buprenorphin, Sufentanil, Hydromorphon, Morphin, Fentanyl, Dextropropoxyphen, Ethylmorphin, Meptazinol, Nalbuphin, Pethidin, Tilidin, Butorphanol, Dextromoramid, Dezocin, Ketobemidon, Oxymorphon, Pentazocin, Diacetylmorphin, Diamorphin, Oxycodon, Alfentanil, Remifentanil, Phenoperidin, Anileridin, Piritramid, Bezitramid, Methadon, Phenazocin, Tapentadol, Axomadol und Tramadol, nichtopioid-Analgetika wie beispielsweise Epibatidin und Scopolamin, Oxicame wie Meloxicam oder Piroxicam, 4-Aminophenol-Derivate wie Flupirtin und Ketamin, Adrenozeptor-Agonisten wie beispielsweise Clonidin, Naphazolin, Tetryzolin, Tramazolin, Xylometazolin und Oxymetazolin, Alphablocker wie beispielsweise Alfuzosin, Bunazosin, Doxazosin, Prazosin, Silodosin, Tamsulosin, Terazosin, Trimazosin und Dapiprazol, Beta-Blocker wie beispielsweise Bupranolol, Timolol, Carvedilol, Sotalol und Nadolol, Parasympathomimetika wie beispielsweise Carbachol, Pilocarpin, Physostigmin, Rivastigmin, Pyridostigmin, Neostigmin oder Distigmin, Aromatasehemmer wie beispielsweise Anastrozol, Letrozol, Exemestan und Formestan, Dopaminagonisten wie beispielsweise Cabergolin, Tergurid, Melevodopa, Pergolid, Lisurid, Rotigotin, Apomorphin, Pramipexol und Ropinirol, Corticosteroide wie beispielsweise natürliche Estrogene wie Estradiol-(17β)-17-butyrylacetat, Estradiol 17-β-cipionat, Estradiol 13,17-β-dienantat, Estradiol 3,17-β-dipropionat, Estradiol Enanthat, Estradiol-3-hydrogensulfat, Estradiol-17-β-(3-phenylpropionat), Estradiolundecylat, Estradiolvalerat, Estradiol-17-(oxohexonat), Epimestrol, Quinestrol, Quinestradol, Ethinylestradiol, Fosferol, Estriol, Lynestrenol, Norethisteron, Hydroxyprogesteron, Progesteron, Norelgestromin, Medroxyprogesteron, Gestronol, Dydrogesteron, Chlormadinon, Allylestrenol, Megestrol und Medrogeston, Antidepressiva wie Clomipramin, Amitriptylin, Amitriptylinoxid, Opipramol, Nortriptylin, Fluvoxamin, Fluoxetin, Citalopram, Escitalopram, Paroxetin, Duloxetin, Bupropion, Selegilin, Rasagilin und Ketamin, Neuroleptika wie beispielsweise Chlorpromazin, Fluphenazin, Levomepromazin, Quetiapin, Zotepin, Bromperidol, Risperidon und Aripiprazol, und Antidementiva wie Donepezil, Rivastigmin, Galantamin und Memantin.

Bevorzugte TDS mit einem erfindungsgemäßen Overtape, welche zumindest einen Wirkstoff enthalten, können dabei zur Schmerztherapie, bei der Behandlung von Asthma bronchiale, Chronisch obstruktiver Lungenerkrankung (COPD), Herzrhythmusstörungen, Angina pectoris, Aufmerksamkeitsdefizit-/Hyperaktivitätsstörung (ADHS), Muskelschwäche, Darmverschluss, des zentralen anticholinergen Syndroms, Darm- und Blasenatonie, Motilitätsstörungen beispielsweise des Darmtrakts, Mundtrockenheit, Intoxikation mit Nervengas, des Restless-Leg Syndroms, Drogenabhängigkeit, Hypertonie, Tachykardie, Herzinsuffizienz, Alzheimer, Demenz, Morbus Parkinson, Amenorrhö, Hyperprolaktinämie, als Brechmittel, zum Abstillen in der Stillzeit, erektiler Dysfunktion, Diabetes, Osteoporose, Hypothyreose, Hyperthyreose, Akromegalie, hormonabhängiger Tumoren, Arthrose, Rheuma, Hyperandrogenämie, Hirsutismus, zur Empfängnisverhütung und zur Therapie bei unerfülltem Kinderwunsch, Depressionen, Angst- und Panikstörungen, Schlafstörungen, Enuresis nocturna, Wahnvorstellungen, Halluzinationen und psychotischen Störungen eingesetzt werden.

Gemäß einer bevorzugten Ausführungsform enthält ein TDS mit einem erfindungsgemäßen Overtape zumindest einen Wirkstoff aus der Klasse der Analgetika, Sympathomimetika oder Aromatasehemmer, besonders bevorzugt aus der Klasse der Opioid-Analgetika und/oder Aromatasehemmer, insbesondere bevorzugt aus der Klasse der Opioid-Analgetika.

Insbesondere wird ein TDS mit einem erfindungsgemäßen Overtape zur Therapie von Drogenabhängigkeit, Hypertonie, Demenzerkrankungen, wie beispielsweise Alzheimer, oder von Schmerz, besonders bevorzugt zur Therapie von Hypertonie und/oder von Schmerz, insbesondere bevorzugt zur Therapie von Schmerz, verwendet.

Gemäß einer besonders bevorzugten Ausführungsform ist der zumindest eine Wirkstoff ausgewählt aus Buprenorphin, Fentanyl, Clonidin, Anastrozol und/oder Rivastigmin, insbesondere aus Buprenorphin, Clonidin und/oder Anastrozol, insbesondere bevorzugt aus Buprenorphin.

Prinzipiell kann der Gehalt des Wirkstoffs in vorteilhaften TDS in einem breiten Bereich variieren. Eine vorteilhafte Wirkstoffmenge, bevorzugt von Analgetika, Sympathomimetika oder Aromatasehemmer, besonders bevorzugt von Sympathomimetika und/oder Opioid-Analgetika, insbesondere bevorzugt von Buprenorphin, liegt bei etwa 0,3 Gew.-% bis etwa 50 Gew.-%, vorzugsweise bei etwa 3 Gew.-% bis etwa 20 Gew.-%, besonders bevorzugt bei etwa 4,5 Gew.-% bis etwa 15 Gew.-%, insbesondere bei etwa 7 Gew.-% bis etwa 12,5 Gew.-%.

Der zumindest eine Wirkstoff, insbesondere Clonidin und/oder Buprenorphin, liegt dabei bevorzugt in Form der freien Base in der wirkstoffhaltigen Matrix vor.

Die wirkstoffhaltige Matrix gibt bei deren Anwendung den zumindest einen Wirkstoff aus der wirkstoffhaltigen Matrix an das umgebende Gewebe ab, wobei im Allgemeinen ein wesentlicher Teil des Wirkstoffs systemisch aufgenommen wird.

Die in der wirkstoffhaltigen Matrix enthaltene absolute Wirkstoffmenge bestimmt üblicherweise die Zeitspanne, in welcher eine kontinuierliche Zufuhr des Wirkstoffs in oder an den Organismus aufrechterhalten wird. Deshalb ist eine möglichst hohe Beladung der wirkstoffhaltigen Matrix mit zumindest einem Wirkstoff dann wünschenswert, wenn die Applikationszeit des TDS lang ist, d. h. mehrere Tage, bevorzugt drei bis sieben Tage, insbesondere vier, fünf, sechs oder sieben Tage, beträgt. Hierbei kann ein erfindungsgemäßes Overtape insbesondere bei einer langen Therapiedauer eine gute Fixierung des TDS auf der Haut und somit eine gute, kontinuierliche Wirkstoffabgabe gewährleisten.

Um eine Migration von Wirkstoff aus dem wirkstoffhaltigen Kern in die wirkstofffreie Matrix des Overtapes zu vermeiden, umfasst ein bevorzugter TDS-Kern (bzw. ein bevorzugtes herkömmlichs TDS, welches mit dem Overtape eingesetzt werden soll) vorteilhaft eine optionale Trennschicht, welche zwischen der wirkstoffhaltigen Matrix und dem Overtape angeordnet ist. Dabei ist diese Trennschicht in einer besonders bevorzugten Ausführungsform wirkstoffundurchlässig ausgestaltet.

Als derartige Trennschichten werden bevorzugt Folien aus beispielsweise Polyester mit einer Stärke von bis zu 200 µm, vorzugsweise aus dem Bereich von 5 bis 150 µm, besonders bevorzugt aus dem Bereich von 7,5 bis 100 µm, insbesondere aus dem Bereich von 10 bis 50 µm, insbesondere bevorzugt aus dem Bereich von 12 bis 30 µm, verwendet. Solche Trennschichten sind flexibel und können sich gegebenenfalls um die Ränder der Matrixschicht, d.h. um die in laterale Richtungen weisenden Seitenflächen der wirkstoffhaltigen Matrix, legen und diese abdecken.

Bevorzugt basiert eine Trennschicht, insbesondere eine wirkstoffundurchlässige Trennschicht, auf einem Polymer ausgewählt aus der Gruppe bestehend aus Polyolefinen, Olefin-Copolymerisaten, Polyestern, Co-Polyestern, Polyamiden, Co-Polyamiden, Polyurethanen und dergleichen. Dabei kann eine Trennschicht sowohl eine Schicht als auch mehreren Schichten aufweisen, welche jeweils aus gleichen oder aus unterschiedlichen Materialien bestehen können. Bevorzugt weist eine Trennschicht jedoch nur eine Schicht auf.

Als Beispiele für geeignete Materialien für eine Trennschicht können Polyester und hiervon insbesondere Polyethylenterephthalate (PET) sowie Polycarbonate genannt werden, Polyolefine wie z. B. Polyethylene, Polypropylene oder Polybutylene, Polyethylenoxide, Polyurethane, Polystyrole, Polyamide, Polyimide, Polyvinylacetate, Polyvinylchloride, Polyvinylidenchloride, Co-Polymerisate wie beispielsweise Acrylnitril-Butadien-Styrol-Terpolymere, oder Ethylen-Vinylacetat-Copolymerisate. Ein bevorzugtes Material einer Trennschicht ist ausgewählt aus einem Polyester, besonders bevorzugt aus einem Polyethylenterephthalat. Eine solche Trennschicht kann beispielsweise unter der Bezeichnung Hostaphan MN 15 DMF von der Firma Mitsubishi Polyester Film GmbH (Deutschland) erworben werden.

Bei einer Ausführungsform eines bevorzugten TDS kann wie oben erwähnt die wirkstoffhaltige Matrix bzw. der TDS-Kern und der darüber hinausragende Teil eines erfindungsgemäßen Overtapes applikationsseitig mit einer in der Fachsprache auch als Release Liner bezeichneten abziehbaren Schutzfolie abgedeckt sein. Bei der Aufbewahrung des TDS wird hierdurch die wirkstoffhaltige Matrix bzw. die Applikationsschicht vor mechanischen Einflüssen und/oder einem unerwünschten Luftzutritt wirksam geschützt. Durch die Unterbindung eines unerwünschten Luftzutritts wird einer Zersetzung von in der Matrix enthaltenen Wirkstoffen, insbesondere von sauerstoffempfindlichen Wirkstoffen, vorgebeugt und so die Lager- bzw. Langzeitstabilität des Pflasters verbessert. Zur Applikation des Pflasters wird dann vor der Befestigung des Systems auf der Haut zunächst die Schutzfolie des Pflasters abgezogen. Zum besseren Greifen und um dadurch ein Ablösen der Schutzfolie zu erleichtern, ragt die Schutzfolie bei vorteilhaften Pflastern über den Rand des Overtapes hinaus.

Geeignete Materialien für einen solchen Release Liner sind beispielsweise Polyester, Polypropylen, Polyvinylchlorid, Aluminium und Papier, wobei zumindest die mit der Matrix in Kontakt kommende Seite des Release Liners eine Siliconbeschichtung, Polyethylenbeschichtung, Fluorsiliconbeschichtung oder Polytetrafluorethylenbeschichtung aufweist.

Wie mehrfach erwähnt, wird ein erfindungsgemäßes Overtape insbesondere für ein transdermales Darreichungssystem verwendet. Dabei weist ein vorteilhaftes Verfahren zur Herstellung eines TDS mit dem erfindungsgemäßen Overtape folgende Schritte auf:
(i) Bereitstellen zumindest einer wirkstoffhaltigen Matrix,
(ii) optional Aufbringen einer Trennschicht auf die zumindest eine wirkstoffhaltige Matrix,
(iii) Stanzen von wirkstoffhaltigen TDS-Kernen aus der wirkstoffhaltigen Matrix und der optionalen Trennschicht,
(iv) Aufbringen des Overtapes gemäß der vorliegenden Erfindung auf die vorstehend erhaltenen TDS-Kerne zum Erhalt eines Gesamt-Laminats,
(v) optional Aufbringen des vorstehend erhaltenen Gesamt-Laminats auf einen Release Liner,
(vi) Stanzen des Gesamt-Laminats und des optionalen Release Liners zum Erhalt von transdermalen Darreichungssystemen,
(vii) optional Verpacken der transdermalen Darreichungssysteme.

Hierbei kann die zumindest eine wirkstoffhaltige Matrix bereits mit einer bedeckenden Schutzfolie versehen sein, welche im weiteren Verlauf der Herstellung in einem oder mehreren Herstellungsschritten durch eine alternative Schutzfolie oder durch einen Release Liner ersetzt werden kann.

Um die gewünschte Scherfestigkeit, Klebkraft und Trennkraft zu erreichen, hat es sich als vorteilhaft erwiesen, dass ein zur Herstellung der wirkstofffreien haftklebenden Matrix eingesetztes quervernetztes Polymer eines bevorzugten erfindungsgemäßen Overtapes eine Brookfield-Viskosität von etwa 1500 bis etwa 4000 mPas, bevorzugt von etwa 2000 bis etwa 3000 mPas, insbesondere bevorzugt von etwa 2300 bis etwa 2700 mPas, aufweist.

Ein zur Herstellung der wirkstofffreien haftklebenden Matrix eingesetztes nicht-quervernetztes Polymer weist bevorzugt eine Brookfield-Viskosität von etwa 2000 bis etwa 6000 mPas, bevorzugt von etwa 3500 bis etwa 5500 mPas, insbesondere bevorzugt von etwa 4300 bis etwa 5000 mPas, auf.

Schließlich betrifft die vorliegende Erfindung auch ein Kit umfassend ein erfindungsgemäßes Overtape und ein TDS, welche in diesem Fall bevorzugt in getrennten Packungseinheiten wie beispielsweise in Beuteln verpackt sind.

Weitere Merkmale der Erfindung ergeben sich aus der nachfolgenden Beschreibung von Ausführungsbeispielen in Verbindung mit den Ansprüchen sowie der Figur. Es sei darauf hingewiesen, dass die Erfindung nicht auf die Ausführungsformen der beschriebenen Ausführungsbeispiele beschränkt, sondern durch den Umfang der beiliegenden Patentansprüche bestimmt ist. Insbesondere können die einzelnen Merkmale bei erfindungsgemäßen Ausführungsformen in anderer Kombination als bei den untenstehend angeführten Beispielen verwirklicht sein. Bei der nachfolgenden Erläuterung einiger Ausführungsbeispiele der Erfindung wird auf die beiliegenden Figuren Bezug genommen. Es zeigen:
Figur 1 einen Längsschnitt durch ein TDS mit einem ersten Ausführungsbeispiel eines erfindungsgemäßen Overtapes, und
Figur 2 eine beispielhafte Unteransicht eines TDS mit einem weiteren Ausführungsbeispiel eines erfindungsgemäßen Overtapes mit deren relativen Ausmaßen.

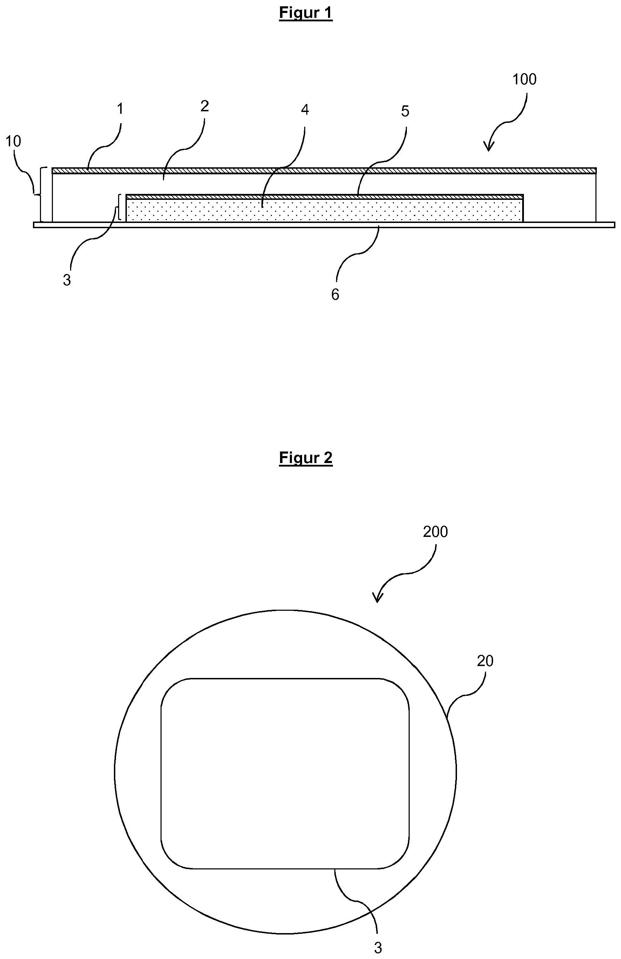

In Figur 1 wird eine erste Ausführungsform eines transdermalen Darreichungssystems 100 (TDS 100) mit einem erfindungsgemäßen Overtape 10 gezeigt. Das TDS 100 weist einen TDS-Kern 3 mit einer wirkstoffhaltigen Schicht 4 und einer Trennschicht 5 auf. Auf der von der wirkstoffhaltigen Schicht 4 abgewandten Seite der Trennschicht 5 befindet sich ein erfindungsgemäßes Overtape 10, welches eine Rückschicht 1 und eine wirkstofffreie Matrix 2 umfasst. Das Overtape 10 überragt den TDS-Kern 3 und somit dessen wirkstoffhaltige Schicht 4 auf allen Seiten. Im vorliegenden Fall sind sowohl das Overtape 10 als auch der TDS-Kern 3 des TDS 100 als rechteckförmige Fläche mit abgerundeten Ecken ausgestaltet (im Schnitt nicht sichtbar). Im Anwendungszustand auf der Haut eines Patienten befindet sich somit rings um die wirkstoffhaltige Schicht 4 des TDS-Kerns 3 die wirkstofffreie Matrix 2 des Overtapes 10. Die wirkstofffreie Matrix 2 des erfindungsgemäßen Overtapes 10 und die wirkstoffhaltige Schicht 4 des TDS-Kerns 3 wird zu deren Schutz von einem Release Liner 6 bedeckt, der vor einer Verwendung abgezogen wird.

Figur 2 stellt eine Unteransicht eines transdermalen Darreichungssystems 200 mit einem erfindungsgemäßen Overtape 20 dar, wobei das Overtape 20 selbst rund ausgestaltet ist. Der TDS-Kern 3 ist hier rechteckförmig mit abgerundeten Ecken ausgestaltet.

### Beispiele

### Beispiel 1:

Zur Herstellung eines erfindungsgemäßen Overtapes wird zunächst ein quervernetztes Polyacrylat (DT 387-2054, Henkel, Deutschland) zu einem nicht-quervernetzten Polyacrylat (DT 387-2051, Henkel, Deutschland) im Verhältnis 1:2 gegeben. Das Gemisch wird anschließend auf eine intermediäre Schutzfolie aufgetragen und getrocknet, sodass ein Laminat mit einer wirkstofffreien haftklebenden Matrix mit einem Flächengewicht der wirkstofffreien haftklebenden Matrix von 60 g/m² erhalten wird. Schließlich wird eine Rückschicht aus Polyolefin-Schaummaterial mit einer Dicke von etwa 0,4 mm auf die getrocknete wirkstofffreie haftklebende Matrix zum Erhalt eines Overtape-Laminats aufkaschiert.

### Beispiel 2:

1 Teil Buprenorphin wird in 2-Propanol suspendiert und mit 13 Teilen eines quervernetzten Polyacrylat-Polymers gemischt. Das so hergestellte Gemisch wird auf eine Schutzfolie aufgetragen, getrocknet und eine wirkstoffhaltige Matrix mit einem Flächengewicht von 50 g/m² erhalten. Im Anschluss wird eine Hostaphan Folie MN 15 DMF (Mitsubishi Polyester Film GmbH, Deutschland) als Trennschicht auf das getrocknete Wirkstofflaminat aufkaschiert. Dieses Wirkstofflaminat wird im vorliegenden Beispiel in TDS-Kerne gestanzt. Im Anschluss wird die intermediäre Schutzfolie des Overtape-Laminats gemäß Beispiel 1 entfernt und das Overtape-Laminat auf die TDS-Kerne zum Erhalt eines Gesamt-Laminats aufkaschiert. Aus diesem Gesamt-Laminat werden nachfolgend TDS ausgestanzt und in Beutel verpackt.

In Tabelle 1 sind Ausmaße und Wirkstoffgehalt verschiedener beispielhafter TDS mit erfindungsgemäßen Overtapes dargestellt.

**Tabelle 1: Ausmaße und Wirkstoffgehalt von TDS mit erfindungsgemäßen Overtapes.**

| | **Buprenorphin TDS 1** | **Buprenorphin TDS 2** | **Buprenorphin TDS 3** |
|---|---|---|---|
| **TDS mit Overtape [mm]** | 72x122 | 72x97 | 72x72 |
| **Wirkstoffkern [mm]** | 50x100 | 50x75 | 50x50 |
| **Wirkstoff [mg]** | 40 | 30 | 20 |
| **Kernfläche [cm²]** | 50 | 37,5 | 25 |
| **Overtape-Fläche [cm²]** | 87,8 | 69,8 | 51,8 |
| **Verhältnis Overtape-Fläche zu Kernfläche** | 1,8 | 1,9 | 2,1 |
| **Klebefläche Overtape (Overtape minus Kern) [cm²]** | 37,8 | 32,3 | 26,8 |

### Beispiel 3:

Overtape-Laminate werden analog zu Beispiel 1 mit einem Flächengewicht der wirkstofffreien Matrix von 50 g/m² hergestellt und hinsichtlich Scherung und Klebkraft untersucht. Wie aus Tabelle 2 ersichtlich, steigt die Scherfestigkeit bei steigenden Anteilen des quervernetzten Polyacrylats (DT 387-2054, Henkel, Deutschland) (Nr. 1-5). Mit steigendem Gehalt des Vernetzungsmittels Aluminium-Acetylacetonat (AlAcAc) (Merck, Deutschland) steigt die Scherfestigkeit bei Anwendung einer konstanten Kraft von 6 N, hingegen verringert sich die Klebkraft (Nr. 6-7).

**Tabelle 2: Scherfestigkeit, Klebkraft und Trennkraft in Abhängigkeit der unterschiedlichen Polymergehalte.**

| Nr. | Feststoffgehalt | | | Scherfestigkeit | | | | | Klebkraft [N/cm] |
|---|---|---|---|---|---|---|---|---|---|
| | DT 387-2051 | DT 387-2054 | AlAcAc | n= | Ablösezeit [s] | Rel. Standardabweichung [%] | Zeit bis zu 2 mm Dehnung [s] | Rel. Standardabweichung [%] | |
| 1 | 80% | 20% | - | 3 | 73 | 3,7 | 39 | 6,2 | 0,81 |
| 2 | 70% | 30% | - | 3 | 138 | 2,8 | 73 | 4,4 | 1,21 |
| 3 | 60% | 40% | - | 3 | 356 | 2,7 | 176 | 8,5 | 0,89 |
| 4 | 55% | 45% | - | 3 | 419 | 4,6 | 230 | 3,7 | 0,96 |
| 5 | 50% | 50% | - | 3 | 694 | 12,2 | 447 | 2,7 | 0,93 |
| 6 | 100% | 0% | 0,1% | 4 | 69 | 10,7 | 34 | 12,3 | 0,95 |
| 7 | 100% | 0% | 0,2% | 6 | 325 | 6,5 | 199 | 10,6 | 0,71 |

## Patentansprüche

1. Overtape (10, 20) umfassend
(a) eine wirkstofffreie haftklebende Matrix (2) und
(b) eine Rückschicht (1),
wobei die wirkstofffreie haftklebende Matrix (2) eine Mischung aus zumindest einem quervernetzten Polymer und zumindest einem nicht-quervernetzten Polymer umfasst, wobei das quervernetzte Polymer ausgewählt ist aus der Gruppe der Polyacrylate, insbesondere aus der Gruppe der Acrylat/Vinylacetat-Copolymere,
wobei das nicht-quervernetzte Polymer ausgewählt ist aus der Gruppe der Polyacrylate, insbesondere aus der Gruppe der Acrylat/Vinylacetat-Copolymere.

2. Overtape (10, 20) gemäß Anspruch 1, wobei das Polyacrylat zwei oder mehrere Monomer-Einheiten umfasst, wobei die Monomere aus der Gruppe ausgewählt sind von Acrylsäure, Vinylacetat, n-Butylacrylat, iso-Butylacrylat, Propylacrylat, Methylacrylat, Tetramethylsuccinonitril, 2-Ethylhexylacrylat, 2-Hydroxyethylacrylat und 2-Hydroxyethylmethacrylat.

3. Overtape (10, 20) gemäß einem der vorhergehenden Ansprüche, wobei das Verhältnis der Gewichte eines quervernetzten Polymers zu einem nicht-quervernetzten Polymer in der getrockneten Matrix in einem Bereich von etwa 1:4 bis etwa 4:1, bevorzugt in einem Bereich von etwa 1:2 bis etwa 2:1, insbesondere in einem Bereich von etwa 1:1,5 bis etwa 1,5:1, insbesondere bevorzugt in einem Bereich von etwa 1:1, liegt.

4. Overtape (10, 20) gemäß einem der vorhergehenden Ansprüche, wobei die Rückschicht (1) ausgewählt ist aus Polyester und/oder Polyolefin, bevorzugt aus einem Polyolefin-Schaummaterial.

5. Overtape gemäß einem der vorhergehenden Ansprüche, wobei die Schichtdicke der getrockneten wirkstofffreien haftklebenden Matrix des Overtapes in einem Bereich von etwa 10 µm bis etwa 500 µm, bevorzugt von etwa 20 µm bis etwa 250 µm, insbesondere von etwa 30 µm bis etwa 200 µm, liegt.

6. Transdermales Darreichungssystem (100, 200) umfassend
(a) das Overtape (10, 20) gemäß einem der Ansprüche 1 bis 5,
(b) einen TDS-Kern (3) umfassend eine wirkstoffhaltige Matrix (4) und optional eine Trennschicht (5),
und
(c) optional einen Release Liner (6),
wobei das Overtape (10, 20) auf allen Seiten der wirkstoffhaltigen Matrix (4) über die wirkstoffhaltige Matrix (4) bzw. die optionale Trennschicht (5) hinausragt.

7. Transdermales Darreichungssystem (100, 200) gemäß Anspruch 6, wobei die Trennschicht (5) ausgewählt ist aus der Gruppe der Polyester, bevorzugt aus Polyethylenterephtalat, Polyvinylchlorid, Polyvinylidenchlorid, Polyacrylonitril oder deren Copolymere.

8. Transdermales Darreichungssystem (100, 200) gemäß Anspruch 6 oder 7, wobei die über die wirkstoffhaltige Matrix (4) und/oder die optionale Trennschicht (5) des TDS-Kerns (3) hinausragende Fläche des Overtapes (10, 20) zumindest dem 0,5-fachen, bevorzugt zumindest dem 0,7-fachen, insbesondere zumindest dem 0,8-fachen, der an der Haut aufliegenden Fläche der wirkstoffhaltigen Matrix (4) und/oder der optionalen Trennschicht (5) des TDS-Kerns (3) entspricht.

9. Transdermales Darreichungssystem (100, 200) gemäß einem der Ansprüche 6 bis 8 zur Verwendung bei der medizinischen oder tiermedizinischen Behandlung.

10. Verwendung eines transdermales Darreichungssystem (100, 200) gemäß einem der Ansprüche 6 bis 8 zur kosmetischen Anwendung.

11. Transdermales Darreichungssystem (100, 200) gemäß einem der Ansprüche 6 bis 8 zur Anwendung in der Schmerztherapie, bei der Behandlung von Hypertonie, Tachykardie, Alzheimer, Demenz, der Parkinson-Erkrankung oder zur Hormonbehandlung, bevorzugt zur Anwendung in der Schmerztherapie,
wobei der Wirkstoff aus der Klasse der Analgetika, Sympathomimetika, Parasympathomimetika, Hormone und/oder Aromatasehemmer ausgewählt ist, bevorzugt aus Opioiden, Dopaminagonisten, Cholinesteraseinhibitoren, Aromatasehemmern, Sexualhormonen und/oder Corticosteroiden, insbesondere aus Buprenorphin, Clonidin, Anastrozol, Letrozol und/oder Exemestan, insbesondere bevorzugt aus Buprenorphin oder Clonidin.

12. Verfahren zur Herstellung eines Overtapes (10, 20), umfassend die Schritte
(i) Bereitstellen zumindest einer wirkstofffreien haftklebenden Matrix (2) enthaltend eine Mischung aus zumindest einem quervernetzten Polymer und zumindest einem nicht-quervernetzten Polymer,
(ii) Aufbringen einer Rückschicht (1) auf die wirkstofffreie haftklebende Matrix (2) zum Erhalt eines Overtape-Laminats,
wobei das quervernetzte Polymer ausgewählt ist aus der Gruppe der Polyacrylate, insbesondere aus der Gruppe der Acrylat/Vinylacetat-Copolymere,
wobei das nicht-quervernetzte Polymer ausgewählt ist aus der Gruppe der Polyacrylate, insbesondere aus der Gruppe der Acrylat/Vinylacetat-Copolymere.

13. Verfahren zur Herstellung eines Overtapes (10, 20) gemäß Anspruch 12, wobei das zur Herstellung der wirkstofffreien haftklebenden Matrix (2) eingesetzte quervernetzte Polymer eine Brookfield-Viskosität von etwa 1500 bis etwa 4000 mPas, bevorzugt von etwa 2000 bis etwa 3000 mPas, insbesondere bevorzugt von etwa 2300 bis etwa 2700 mPas, aufweist.

14. Verfahren zur Herstellung eines Overtapes (10, 20) gemäß Anspruch 12 oder 13, wobei das zur Herstellung der wirkstofffreien haftklebenden Matrix (2) eingesetzte nicht-quervernetzte Polymer eine Brookfield-Viskosität von etwa 2000 bis etwa 6000 mPas, bevorzugt von etwa 3500 bis etwa 5500 mPas, insbesondere bevorzugt von etwa 4300 bis etwa 5000 mPas, aufweist.

15. Verfahren zur Herstellung eines transdermalen Darreichungssystems (100, 200), wobei das Verfahren folgende Schritte umfasst:
(i) Bereitstellen zumindest einer wirkstoffhaltigen Matrix (4),
(ii) optional Aufbringen einer Trennschicht (5) auf die zumindest eine wirkstoffhaltige Matrix (4),
(iii) Stanzen von wirkstoffhaltigen TDS-Kernen (3) aus der wirkstoffhaltigen Matrix (4) und der optionalen Trennschicht (5),
(iv) Aufbringen eines Overtapes (10, 20) gemäß einem der Ansprüche 1 bis 5 auf die vorstehend erhaltenen wirkstoffhaltigen TDS-Kerne (3) zum Erhalt eines Gesamt-Laminats,
(v) optional Aufbringen des vorstehend erhaltenen Gesamt-Laminats auf einen Release Liner (6),
(vi) Stanzen des Gesamt-Laminats und des optionalen Release Liners (6) zum Erhalt von transdermalen Darreichungssystemen (100, 200),
(vii) optional Verpacken der transdermalen Darreichungssysteme (100, 200).

## Claims

1. An overtape (10, 20) comprising
(a) an active-ingredient-free pressure-sensitive adhesive matrix (2) and
(b) a backing layer (1),
wherein the active-ingredient-free pressure-sensitive adhesive matrix (2) comprises a mixture of at least one crosslinked polymer and at least one non-crosslinked polymer, wherein the crosslinked polymer is selected from the group of polyacrylates, in particular from the group of acrylate/vinyl acetate copolymers,
wherein the non-crosslinked polymer is selected from the group of polyacrylates, in particular from the group of acrylate/vinyl acetate copolymers.

2. The overtape (10, 20) according to claim 1, wherein the polyacrylate comprises two or more monomer units, wherein the monomers are selected from the group of acrylic acid, vinyl acetate, n-butyl acrylate, iso-butyl acrylate, propyl acrylate, methyl acrylate, tetramethylsuccinonitrile, 2-ethylhexyl acrylate, 2-hydroxyethyl acrylate and 2-hydroxyethyl methacrylate.

3. The overtape (10, 20) according to any one of the preceding claims, wherein the ratio of the weights of a crosslinked polymer to a non-crosslinked polymer in the dried matrix lies in a range of from approximately 1:4 to approximately 4:1, preferably in a range of from approximately 1:2 to approximately 2:1, in particular in a range of from approximately 1:1.5 to approximately 1.5:1, in particular preferably in a range of approximately 1:1.

4. The overtape (10, 20) according to any one of the preceding claims, wherein the backing layer (1) is selected from polyester and/or polyolefin, preferably from a polyolefin foam material.

5. The overtape according to any one of the preceding claims, wherein the layer thickness of the dried active-ingredient-free pressure-sensitive adhesive matrix of the overtape lies in a range of from approximately 10 µm to approximately 500 µm, preferably of from approximately 20 µm to approximately 250 µm, in particular of from approximately 30 µm to approximately 200 µm.

6. A transdermal administration system (100, 200) comprising
(a) the overtape (10, 20) according to any one of claims 1 to 5,
(b) a TDS core (3) comprising an active-ingredient-containing matrix (4) and optionally a separating layer (5),
and
(c) optionally a release liner (6),
wherein the overtape (10, 20) extends beyond the active-ingredient-containing matrix (4) or the optional separating layer (5) on all sides of the active-ingredient-containing matrix (4).

7. The transdermal administration system (100, 200) according to claim 6, wherein the separating layer (5) is selected from the group of polyesters, preferably from polyethylene terephthalate, polyvinylchloride, polyvinylidene chloride, polyacrylonitrile or copolymers thereof.

8. The transdermal administration system (100, 200) according to claim 6 or 7, wherein the area of the overtape (10, 20) extending beyond the active-ingredient-containing matrix (4) and/or the optional separating layer (5) of the TDS core (3) corresponds to at least 0.5 times, preferably to at least 0.7 times, in particular to at least 0.8 times the area of the active-ingredient-containing matrix (4) resting against the skin and/or of the optional separating layer (5) of the TDS core (3).

9. The transdermal administration system (100, 200) according to any one of claims 6 to 8 for use in medical or veterinary treatment.

10. Use of a transdermal administration system (100, 200) according to any one of claims 6 to 8 for cosmetic application.

11. The transdermal administration system (100, 200) according to any one of claims 6 to 8 for use in pain therapy, in the treatment of hypertension, tachycardia, Alzheimer's disease, dementia, Parkinson's disease or for hormone treatment, preferably for use in pain therapy,
wherein the active ingredient is selected from the class of analgesics, sympathomimetics, parasympathomimetics, hormones and/or aromatase inhibitors, preferably from opioids, dopamine agonists, cholinesterase inhibitors, aromatose inhibitors, sexual hormones and/or corticosteroids, in particular from buprenorphine, clonidine, anastrozole, letrozole and/or exemestane, in particular preferably from buprenorphine or clonidine.

12. A method for producing an overtape (10, 20), comprising the steps of
(i) providing at least one active-ingredient-free pressure-sensitive adhesive matrix (2) containing a mixture of at least one crosslinked polymer and at least one non-crosslinked polymer,
(ii) applying a backing layer (1) to the active-ingredient-free pressure-sensitive adhesive matrix (2) to obtain an overtape laminate,
wherein the crosslinked polymer is selected from the group of polyacrylates, in particular from the group of acrylate/vinyl acetate copolymers,
wherein the non-crosslinked polymer is selected from the group of polyacrylates, in particular from the group of acrylate/vinyl acetate copolymers.

13. The method for producing an overtape (10, 20) according to claim 12, wherein the crosslinked polymer used to produce the active-ingredient-free pressure-sensitive adhesive matrix (2) has a Brookfield viscosity of from approximately 1500 to approximately 4000 mPas, preferably of from approximately 2000 to approximately 3000 mPas, in particular preferably of from approximately 2300 to approximately 2700 mPas.

14. The method for producing an overtape (10, 20) according to claim 12 or 13, wherein the non-crosslinked polymer used to produce the active-ingredient-free pressure-sensitive adhesive matrix (2) has a Brookfield viscosity of from approximately 2000 to approximately 6000 mPas, preferably of from approximately 3500 to approximately 5500 mPas, in particular preferably of from approximately 4300 to approximately 5000 mPas.

15. A method for producing a transdermal administration system (100, 200), wherein the method comprises the following steps:
(i) providing at least one active-ingredient-containing matrix (4),
(ii) optionally applying a separating layer (5) to the at least one active-ingredient-containing matrix (4),
(iii) die-cutting active-ingredient-containing TDS cores (3) from the active-ingredient-containing matrix (4) and the optional separating layer (5),
(iv) applying an overtape (10, 20) according to any one of claims 1 to 5 to the above obtained active-ingredient-containing TDS cores (3) to obtain an overall laminate,
(v) optionally applying the above obtained overall laminate to a release liner (6),
(vi) die-cutting the overall laminate and the optional release liner (6) to obtain transdermal administration systems (100, 200),
(vii) optionally packaging the transdermal administration systems (100, 200).

## Revendications

1. Ruban de recouvrement (10, 20), comprenant
(a) une matrice adhésive sensible à la pression sans principe actif (2) et
(b) une couche arrière (1),
la matrice adhésive sensible à la pression sans principe actif (2) comprenant un mélange d'au moins un polymère réticulé et d'au moins un polymère non-réticulé, le polymère non-réticulé étant choisi dans le groupe des polyacrylates, notamment dans le groupe des copolymères acrylate / acétate de vinyle,
le polymère non-réticulé étant choisi dans le groupe des polyacrylates, notamment dans le groupe des copolymères acrylate / acétate de vinyle.

2. Ruban de recouvrement (10, 20) selon la revendication 1, ledit polyacrylate comprenant deux ou plusieurs unités de monomère, les monomères étant choisis dans le groupe d'acide acrylique, acétate de vinyle, acrylate de n-butyle, acrylate d'iso-butyle, acrylate de propyle, acrylate de méthyle, tétraméthylsuccinonitrile, acrylate de 2-éthylhexyle, acrylate d'hydroxyéthyle et de méthacrylate de 2-hydroxyéthyle.

3. Ruban de recouvrement (10, 20) selon l'une des revendications précédentes, le rapport pondéral entre un polymère réticulé et un polymère non-réticulé dans la matrice séchée étant compris entre environ 1:4 et environ 4:1, de préférence compris entre environ 1:2 et environ 2:1, notamment compris entre environ 1:1,5 et environ 1,5:1, avec une préférence particulière égal à environ 1:1.

4. Ruban de recouvrement (10, 20) selon l'une des revendications précédentes, la couche arrière (1) étant choisie parmi des polyesters et/ou des polyoléfines, de préférence parmi une mousse de polyoléfine.

5. Ruban de recouvrement selon l'une des revendications précédentes, l'épaisseur de couche de la matrice adhésive sensible à la pression sans principe actif du ruban de recouvrement étant comprise, à l'état séché, entre environ 10 µm et environ 500 µm, de préférence entre environ 20 µm et environ 250 µm, notamment entre environ 30 µm et environ 200 µm.

6. Système d'administration transdermique (100, 200) comprenant
(a) le ruban de recouvrement (10, 20) selon l'une des revendications 1 à 5,
(b) un noyau SAT (3) comprenant une matrice contenant du principe actif (4) et optionnellement une couche séparatrice (5),
et
(c) optionnellement, un film support (6),
le ruban de recouvrement (10, 20) s'étend la matrice contenant du principe actif (4) et/ou la couche séparatrice (5) optionnelle sur tous les côtés de la matrice contenant du principe actif (4).

7. Système d'administration transdermique (100, 200) selon la revendication 6, la couche séparatrice (5) étant choisie dans le groupe des polyesters, de préférence parmi le polyéthylène-téréphtalate, le polychlorure de vinyle, le polyacrylonitrile ou leurs copolymères.

8. Système d'administration transdermique (100, 200) selon les revendications 6 ou 7, la surface du ruban de recouvrement (10, 20) qui s'étend la matrice contenant du principe actif (4) et/ou la couche séparatrice optionnelle (5) du noyau SAT (3) correspondant au moins à 0,5 fois, de préférence au moins à 0,7 fois, avec une préférence particulière au moins à 0,8 fois la surface de la matrice contenant du principe actif (4) et/ou de la couche séparatrice optionnelle (5) du noyau SAT (3).

9. Système d'administration transdermique (100, 200) selon l'une des revendications 6 à 8, destiné à être utilisé dans un traitement médical ou vétérinaire.

10. Utilisation d'un système d'administration transdermique (100, 200) selon l'une des revendications 6 à 8 pour une application cosmétique.

11. Système d'administration transdermique (100, 200) selon l'une des revendications 6 à 8, pour une application dans la thérapie analgésique, dans le traitement de l'hypertonie, de la tachycardie, de la maladie d'Alzheimer, de la démence, de la maladie de Parkinson ou dans la thérapie hormonale, préférentiellement dans la thérapie analgésique,
le principe actif étant choisi dans la catégorie des analgésiques, des sympathicomimétiques, des parasympathicomimétiques, des hormones et/ou des inhibiteurs d'aromatase, préférentiellement parmi les opioïdes, les agonistes de dopamine, les inhibiteurs de cholinestérase, les inhibiteurs d'aromatase, les hormones sexuelles, et/ou les corticostéroïdes, notamment parmi la buprénorphine, la clonidine, l'anastrozole, le létrozole et/ou l'exémestane, avec une préférence particulière parmi la buprénorphine ou la clonidine.

12. Procédé de fabrication d'un ruban de recouvrement (10, 20), comprenant les étapes consistant à
(i) mettre à disposition au moins une matrice adhésive sensible à la pression sans principe actif (2) contenant au moins un polymère réticulé et au moins un polymère non-réticulé,
(ii) appliquer une couche arrière (1) sur la matrice adhésive sensible à la pression sans principe actif (2) pour ainsi obtenir un ruban de recouvrement stratifié,
le polymère réticulé étant choisi dans le groupe des polyacrylates, notamment dans le groupe des copolymères acrylate / acétate de vinyle
le polymère non-réticulé étant choisi dans le groupe des polyacrylates, notamment dans le groupe des copolymères acrylate / acétate de vinyle.

13. Procédé de fabrication d'un ruban de recouvrement (10, 20) selon la revendication 12, le polymère réticulé, mis en œuvre pour réaliser la matrice adhésive sensible à la pression sans principe actif (2), ayant une viscosité Brookfield d'environ 1500 à environ 4000 mPas, préférentiellement d'environ 2000 à environ 3000 mPas, avec une préférence particulière d'environ 2300 à environ 2700 mPas.

14. Procédé de fabrication d'un ruban de recouvrement (10, 20) selon les revendications 12 ou 13, le polymère non-réticulé, mis en œuvre pour réaliser la matrice adhésive sensible à la pression sans principe actif (2), ayant une viscosité Brookfield d'environ 2000 à environ 6000 mPas, préférentiellement d'environ 3500 à environ 5500 mPas, avec une préférence particulière d'environ 4300 à environ 5000 mPas.

15. Procédé de fabrication d'un système d'administration transdermique (100, 200), le procédé comprenant les étapes suivantes :
(i) mettre à disposition au moins une matrice contenant du principe actif (4),
(ii) optionnellement, appliquer une couche séparatrice (5) sur l'au moins une matrice contenant du principe actif (4),
(iii) réaliser, par poinçonnage pratiqué dans la matrice contenant du principe actif (4) et la couche séparatrice (5) optionnelle, des noyaux SAT (3) contenant du principe actif,
(iv) appliquer un ruban de recouvrement (10, 20) selon l'une des revendications 1 à 5 sur les noyaux SAT (3) contenant du principe actif tels qu'obtenus ci-avant, pour ainsi obtenir un produit stratifié global,
(v) optionnellement, appliquer un film support (6) sur le produit stratifié global tel qu'obtenu ci-avant,
(vi) soumettre le produit stratifié global et la film support (6) optionnelle à un poinçonnage pour ainsi obtenir des systèmes d'administration transdermique (100, 200),
(vii) optionnellement, emballer les systèmes d'administration transdermales (100, 200).
